# FASCICULE DE BREVET EUROPEEN

(11) **EP 0 765 658 B1**
(45) Date de publication et mention de la délivrance du brevet: **19.11.1997**
(21) Numéro de dépôt: 96401970.7
(22) Date de dépôt: 16.09.1996
(51) Int. Cl.: A61K 7/48, A61K 31/00, A61K 31/60

(54) **Utilisation de dérivés d'acide salicylique pour le traitement des vergetures**
Verwendung von Salicylsäure-Derivaten zur Behandlung von kleiner Streiffen auf der Haut
Use of salicylic acid derivatives for the treatment of striae

(30) Priorité: 28.09.1995 FR 9511405
(43) Date de publication de la demande: 02.04.1997
(73) Titulaire: L'OREAL, 75008 Paris (FR)
(72) Inventeur: Fanchon, Chantal, 75015 Paris (FR)
(74) Mandataire: Lhoste, Catherine

(56) Documents cités:
- EP-A- 0 232 982
- EP-A- 0 378 936
- WO-A-93/10756
- US-A- 5 444 091

## Description

L'invention se rapporte l'utilisation de dérivés d'acide salicylique dans ou pour la préparation d'une composition cosmétique ou dermatologique par application topique sur la peau du corps, dans le but de traiter les vergetures.

Les vergetures sont des stries linéaires ou légèrement sineuses séparées par des intervalles de peau saine. Elles seraient dues à une fragilisation locale des tissus sous-cutanés, dont les causes peuvent être variées et n'ont pas toujours été parfaitement identifiées. Les vergetures peuvent apparaître notamment au cours de la puberté ou de la grossesse, ou bien être provoquées par exemple par une importante prise ou perte de poids, par une maladie infectieuse ou par un traitement aux corticoïdes.

D'une façon générale, les vergetures sont considérées comme des cicatrices indélébiles.

Différentes molécules sont préconisées pour éviter la formation des vergetures ou traiter les vergetures formées. Par exemple, le document EP-A-415458 décrit l'utilisation de rétinoïdes et le document US-A-5444091 celle d'alpha-hydroxyacides, et notamment d'acide glycolique.

On cherche à diversifier le type de molécule utilisée pour le traitement des vergetures et il subsiste donc le besoin d'autres substances ayant les propriétés appropriées.

La demanderesse a trouvé de manière inattendue que des dérivés d'acide salicylique présentaient la propriété d'avoir une action sur les vergetures.

Certes, il est connu du document EP-A-378936 d'utiliser des dérivés d'acide salicylique comme agents kératolytiques pour prévenir le vieillissement de la peau. Toutefois, ce document n'enseigne nullement que ces dérivés d'acide salicylique réduisent ou empêchent la formation des vergetures.

Aussi, la présente invention a pour objet l'utilisation, dans et/ou pour la préparation d'une composition cosmétique et/ou dermatologique destinée à prévenir ou à traiter les vergetures, d'un dérivé d'acide salicylique de formule (I) ou d'un sel d'un tel dérivé : 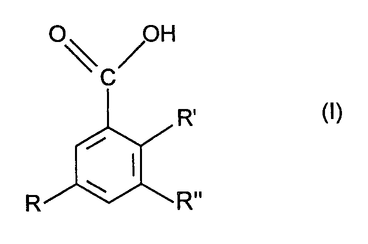 dans laquelle :
R représente un groupement hydroxyle, une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 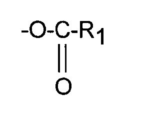 où R₁ est un groupement aliphatique saturé ou insaturé ayant de 1 à 18 atomes de carbone ;
R'' représente un hydrogène, un groupement hydroxyle ou une fonction ester de formule : 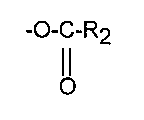 où R₂ est un groupement aliphatique, identique ou différent de R₁, saturé ou insaturé ayant de 1 à 18 atomes de carbone.

Lorsque R'' représente un hydrogène, le dérivé de l'acide salicylique est choisi de façon avantageuse parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique. On peut cependant utiliser aussi les acides 5-tert-octylsalicylique, 3-tert-butyl-5 ou 6-méthylsalicylique, 3,5-diisopropylsalicylique, 5-butoxysalicylique, 5-octyloxysalicylique. On peut également utiliser les composés décrits dans le document EP-A-570230.

Lorsque R ou R'' représente un groupement hydroxyle, le dérivé de l'acide salicylique est choisi de façon avantageuse parmi l'acide gentisique (acide dihydroxy-2,5-benzoïque) et l'acide dihydroxy-2,3-benzoïque. Ces acides peuvent être utilisés tels quels ou sous forme d'extraits naturels en contenant. Comme extrait naturel, on peut citer par exemple la gentiane.

Les acides de formule (I) peuvent se présenter également sous forme de sels, et en particulier sous forme de sels obtenus par salification avec une base.

Comme base susceptible de salifier les dérivés de l'acide salicylique selon l'invention, on peut citer les bases minérales comme les hydroxydes de métaux alcalins (hydroxydes de sodium et de potassium) ou les hydroxydes d'ammonium ou mieux encore les bases organiques.

De préférence, on utilise des bases amphotères pour la salification des dérivés de l'acide salicylique, c'est-à-dire des bases ayant à la fois des groupements fonctionnels anioniques et cationiques.

Les bases amphotères peuvent être des amines organiques primaires, secondaires, tertiaires ou cycliques et plus spécialement des acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine. Ces bases sont utilisées en quantités suffisantes pour amener le pH de l'émulsion entre 5 et 7 et donc proche de celui de la peau. Il s'ensuit une grande compatibilité de la composition de l'invention vis-à-vis de la peau.

Les dérivés d'acide salicylique ou leurs sels sont utilisés, selon la présente invention, de préférence en quantité allant de 0,001 à 20 % en poids et encore plus préférentiellement de 0,1 à 10 %.

L'application d'une composition contenant un dérivé d'acide salicylique selon l'invention permet d'obtenir une nette diminution voire une disparition complète des vergetures.

Aussi, l'invention a encore pour objet un procédé cosmétique pour prévenir et/ou traiter les vergetures, consistant à appliquer sur la peau une composition contenant un dérivé d'acide salicylique de formule (I) ou d'un sel d'un tel dérivé.

Les compositions contenant les dérivés d'acide salicylique selon l'invention peuvent présenter toutes les formes galéniques normalement utilisées pour une application topique, par exemple sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

De façon connue, les compositions cosmétiques ou dermatologiques de l'invention peuvent contenir des adjuvants habituels dans le domaine cosmétique ou dermatologique, tels que les émulsionnants, les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans les domaines cosmétique et/ou dermatologique, et par exemple de 0,01 % à 20 % du poids total de la composition. Ces adjuvants, selon leur nature, peuvent être introduits dans la phase grasse, dans la phase aqueuse et/ou dans les vésicules lipidiques.

Comme émulsionnants, on peut utiliser des émulsionnants eau-dans huile (E/H) ou huile-dans-eau (H/E) selon l'émulsion finale souhaitée.

Comme émulsionnants, on peut citer par exemple le stéarate de PEG-40, le tristéarate de sorbitan et le mélange de stéarate de glycéryl et de stéarate de PEG-30, vendu sous la dénomination « Arlatone 983 » par la société ICI.

Le taux d'émulsionnant peut aller de 0,1 % à 15 % en poids, et de préférence de 0,5 % à 5 % en poids, par rapport au poids total de la composition.

On peut ajouter à la composition selon l'invention des coémulsionnants, par exemple en une quantité allant de 0,05 % à 10 % en poids par rapport au poids total de la composition. Comme coémulsionnant, on peut citer le stéarate de glycérol.

Dans les dispersions de vésicules lipidiques, l'émulsionnant peut être constitué par des vésicules de lipides ioniques et/ou non ioniques.

Comme huiles utilisables dans l'invention, on peut citer les huiles minérales, les huiles végétales (huile de tournesol, huile d'amande d'abricot, huile de karité), les huiles de synthèse (palmitate d'éthyl-hexyle, polyisobutène hydrogéné), les huiles siliconées (cyclométhicone, diméthicone) et les huiles fluorées (perfluoropolyéthers). La phase grasse peut comprendre aussi des alcools gras (alcool cétylique), des acides gras et des cires.

Comme gélifiants hydrophiles, on peut citer les polymères carboxyvinyliques, les polyacrylates ou polyméthacrylates de glycéryle, les polyacrylamides tels que le mélange Polyacrylamide/Isoparaffine/Laureth-7 vendu par la société Seppic sous la dénomination de Sepigel 305, les gommes naturelles (xanthane) et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser par exemple les protéines ou les hydrolysats de protéine, les acides aminés, les polyols, en particulier la glycérine ou le sorbitol, l'urée, l'allantoïne, les sucres et leurs dérivés, l'acide glycyrrhétinique.

Comme actifs lipophiles, on peut citer le tocophérol (vitamine E) et ses dérivés, les acides gras essentiels, les céramides, les huiles essentielles.

Les exemples suivants illustrent l'invention. Dans ces exemples, les proportions indiquées sont des pourcentages en poids. Ces exemples se rapportent à des émulsions huile-dans-eau, obtenues selon les méthodes classiques dans le domaine, c'est-à-dire par introduction, sous agitation et à une température d'environ 70°C, de la phase huileuse contenant l'émulsionnant et les adjuvants lipophiles, dans la phase aqueuse comprenant le gélifiant et les adjuvants hydrophiles, puis refroidissement jusqu'à la température ambiante.

### Exemple 1 : Emulsion H/E

| | |
|---|---|
| Acide gentisique | 10 % |
| Stéarate de glycéryl/stéarate de PEG-30 (Arlatone 983) | 6 % |
| Huile minérale | 3,5 % |
| Octyl dodecanol (solvant) | 6 % |
| Gomme de xanthane | 0,2 % |
| Diméthicone | 1 % |
| Alcool cétylique | 4 % |
| Soude | qsp pH=3 |
| Eau | qsp 100 % |

On obtient une crème blanche qui, par application quotidienne pendant un mois, permet un bon estompage des vergetures.

### Exemple 2 : Emulsion H/E

| | |
|---|---|
| Stéarate de PEG-40 | 2 % |
| Tristéarate de sorbitan | 0,9 % |
| Alcool cétylique | 4 % |
| Stéarate de glycérol | 3 % |
| Polyisobutène hydrogéné | 8 % |
| Palmitate d'éthyl-hexyle | 3,5 % |
| Cyclométhicone | 5 % |
| Glycérine | 3 % |
| Polyacrylamide/Isoparaffine/Laureth-7 (Sepigel 305 vendu par la société Seppic) | 1 % |
| Acide n-octanoyl-5 salicylique | 2 % |
| Glycérine | 5 % |
| Eau | qsp 100 % |

On obtient une crème permettant d'empêcher efficacement la formation des vergetures.

### Exemple 3 : Emulsion H/E

| | |
|---|---|
| Stéarate de PEG-40 | 2 % |
| Huile végétale | 22 % |
| Tristéarate de sorbitan | 0,9 % |
| Monostéarate de glycérol | 3 % |
| Alcool cétylique | 4 % |
| Glycérine | 3 % |
| Acide n-octanoyl-5 salicylique | 2 % |
| Eau | qsp 100 % |

On obtient une crème permettant de Imiter ou d'empêcher la formation des vergetures.

## Revendications

1. Utilisation, pour la préparation d'une composition cosmétique et/ou dermatologique destinée à prévenir ou à traiter les vergetures, d'un dérivé d'acide salicylique de formule (I) ou d'un sel d'un tel dérivé : 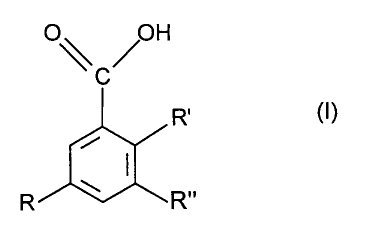 dans laquelle :
R représente un groupement hydroxyle, une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 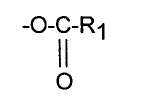 où R₁ est un groupement aliphatique saturé ou insaturé ayant de 1 à 18 atomes de carbone ;
R'' représente un hydrogène, un groupement hydroxyle ou une fonction ester de formule : 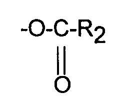 où R₂ est un groupement aliphatique, identique ou différent de R₁, saturé ou insaturé ayant de 1 à 18 atomes de carbone.

2. Utilisation selon la revendication 1, caractérisée en ce que le dérivé d'acide salicylique est choisi parmi les dérivés de formule (I) dans laquelle R'' représente un hydrogène.

3. Utilisation selon la revendication 2, caractérisée en ce que le dérivé d'acide salicylique est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique.

4. Utilisation selon la revendication 3, caractérisée en ce que le dérivé d'acide salicylique est l'acide n-octanoyl-5 salicylique.

5. Utilisation selon la revendication 1, caractérisée en ce que le dérivé d'acide salicylique est choisi parmi les dérivés de formule (I) dans laquelle R ou R'' représente un groupement hydroxyle.

6. Utilisation selon la revendication 5, caractérisée en ce que le dérivé d'acide salicylique est choisi parmi les acides gentisique et dihydroxy-2,3-benzoïque.

7. Utilisation selon l'une quelconque des revendications précédentes, caractérisée en ce que le dérivé d'acide salicylique est sous forme salifiée par une base minérale ou organique.

8. Utilisation selon l'une quelconque des revendications précédentes, caractérisée en ce que le dérivé d'acide salicylique ou son sel est présent en une concentration allant de 0,001 à 20 % en poids par rapport au poids total de la composition.

9. Utilisation selon l'une quelconque des revendications précédentes, caractérisée en ce que la composition est une solution aqueuse, huileuse ou hydroalcoolique, une émulsion eau-dans-huile, une émulsion huile-dans-eau, une microémulsion, un gel aqueux, un gel huileux, un gel anhydre, un sérum, une dispersion de vésicules.

10. Procédé cosmétique pour prévenir et/ou traiter les vergetures, consistant à appliquer sur la peau une composition contenant un dérivé d'acide salicylique de formule (I) ou d'un sel d'un tel dérivé : 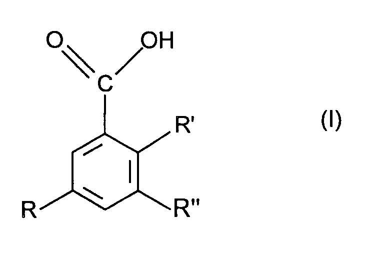 dans laquelle :
R représente un groupement hydroxyle, une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R' représente un groupement hydroxyle ou une fonction ester de formule : 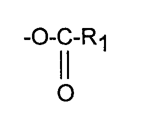 où R₁ est un groupement aliphatique saturé ou insaturé ayant de 1 à 18 atomes de carbone ;
R'' représente un hydrogène, un groupement hydroxyle ou une fonction ester de formule : 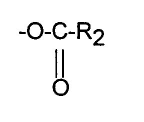 où R₂ est un groupement aliphatique, identique ou différent de R₁, saturé ou insaturé ayant de 1 à 18 atomes de carbone.

11. Procédé selon la revendication 10, caractérisé en ce que le dérivé d'acide salicylique est choisi parmi les dérivés de formule (I) dans laquelle R'' représente un hydrogène.

12. Procédé selon la revendication 11, caractérisé en ce que le dérivé d'acide salicylique est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique.

13. Procédé selon la revendication 12, caractérisée en ce que le dérivé d'acide salicylique est l'acide n-octanoyl-5 salicylique.

14. Procédé selon la revendication 10, caractérisé en ce que le dérivé d'acide salicylique est choisi parmi les dérivés de formule (I) dans laquelle R ou R'' représente un groupement hydroxyle.

15. Procédé selon la revendication 14, caractérisé en ce que le dérivé d'acide salicylique est choisi parmi les acides gentisique et dihydroxy-2,3-benzoïque.

## Claims

1. Use, for the preparation of a cosmetic and/or dermatological composition intended to prevent or treat vibices, of a salicylic acid derivative of formula (I) or a salt of such a derivative: 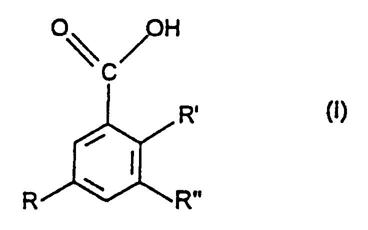 in which:
R represents a hydroxyl group, a linear, branched or cyclized, saturated, aliphatic, alkoxy, ester or ketoxy chain, an unsaturated chain bearing one or more conjugated or non-conjugated double bonds, these chains containing from 2 to 22 carbon atoms and possibly being substituted with at least one substituent chosen from halogen atoms, the trifluoromethyl group, hydroxyl groups in free form or esterified with an acid having from 1 to 6 carbon atoms, or alternatively with a carboxyl function, free or esterified with a lower alcohol having from 1 to 6 carbon atoms;
R' represents a hydroxyl group or an ester function of formula: 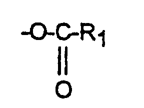 where R₁ is a saturated or unsaturated aliphatic group having from 1 to 18 carbon atoms;
R'' represents a hydrogen, a hydroxyl group or an ester function of formula: 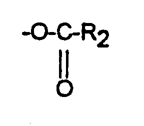 where R₂ is a saturated or unsaturated aliphatic group identical to or different from R₁, having from 1 to 18 carbon atoms.

2. Use according to Claim 1, characterized in that the salicylic acid derivative is chosen from derivatives of formula (I) in which R'' represents a hydrogen.

3. Use according to Claim 2, characterized in that the salicylic acid derivative is chosen from 5-n-octanoylsalicylic acid, 5-n-decanoylsalicylic acid, 5-n-dodecanoylsalicylic acid, 5-n-octylsalicylic acid, 5-n-heptyloxysalicylic acid and 4-n-heptyloxysalicylic acid.

4. Use according to Claim 3, characterized in that the salicylic acid derivative is 5-n-octanoylsalicylic acid.

5. Use according to Claim 1, characterized in that the salicylic acid derivative is chosen from derivatives of formula (I) in which R or R'' represents a hydroxyl group.

6. Use according to Claim 5, characterized in that the salicylic acid derivative is chosen from gentisic acid and 2,3-dihydroxybenzoic acid.

7. Use according to any one of the preceding claims, characterized in that the salicylic acid derivative is in a form salified with an inorganic or organic base.

8. Use according to any one of the preceding claims, characterized in that the salicylic acid derivative or its salt is present in a concentration ranging from 0.001 to 20% by weight relative to the total weight of the composition.

9. Use according to any one of the preceding claims, characterized in that the composition is an aqueous, oily or aqueous-alcoholic solution, a water-in-oil emulsion, an oil-in-water emulsion, a microemulsion, an aqueous gel, an oily gel, an anhydrous gel, a serum or a vesicle dispersion.

10. Cosmetic process for preventing and/or treating vibices, which consists in applying to the skin a composition containing a salicylic acid derivative of formula (I) or a salt of such a derivative: 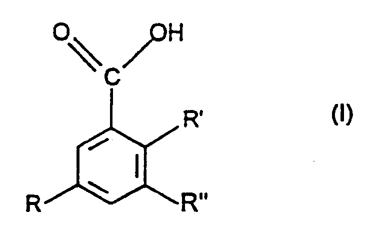 in which:
R represents a hydroxyl group, a linear, branched or cyclized, saturated, aliphatic, alkoxy, ester or ketoxy chain, an unsaturated chain bearing one or more conjugated or non-conjugated double bonds, these chains containing from 2 to 22 carbon atoms and possibly being substituted with at least one substituent chosen from halogen atoms, a trifluoromethyl group, hydroxyl groups in free form or esterified with an acid having from 1 to 6 carbon atoms, or alternatively with a carboxyl function, free or esterified with a lower alcohol having from 1 to 6 carbon atoms;
R' represents a hydroxyl group or an ester function of formula: 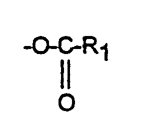 where R₁ is a saturated or unsaturated aliphatic group having from 1 to 18 carbon atoms;
R'' represents a hydrogen, a hydroxyl group or an ester function of formula: 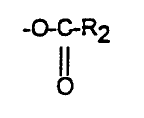 where R₂ is a saturated or unsaturated aliphatic group identical to or different from R₁, having from 1 to 18 carbon atoms.

11. Process according to Claim 10, characterized in that the salicylic acid derivative is chosen from derivatives of formula (I) in which R'' represents a hydrogen.

12. Process according to Claim 11, characterized in that the salicylic acid derivative is chosen from 5-n-octanoylsalicylic acid, 5-n-decanoylsalicylic acid, 5-n-dodecanoylsalicylic acid, 5-n-octylsalicylic acid, 5-n-heptyloxysalicylic acid and 4-n-heptyloxysalicylic acid.

13. Process according to Claim 12, characterized in that the salicylic acid derivative is 5-n-octanoylsalicylic acid.

14. Process according to Claim 10, characterized in that the salicylic acid derivative is chosen from derivatives of formula (I) in which R or R'' represents a hydroxyl group.

15. Process according to Claim 14, characterized in that the salicylic acid derivative is chosen from gentisic acid and 2,3-dihydroxybenzoic acid.

## Patentansprüche

1. Verwendung eines Salicylsäurederivats der Formel I oder eines Salzes dieser Derivate 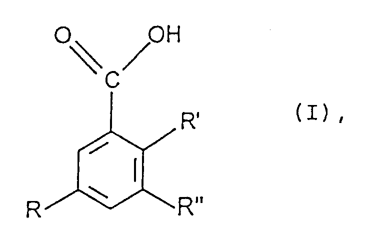 worin bedeuten:
R eine Hydroxygruppe, eine gesättigte, geradkettige, verzweigte oder cyclische aliphatische Gruppe, Alkoxygruppe, Estergruppe oder Acylgruppe und eine ungesättigte Gruppe, die eine oder mehrere ggf. konjugierte Doppelbindungen aufweist, wobei die Gruppen 2 bis 22 Kohlenstoffatome aufweisen und mit mindestens einem Substituenten, der unter Halogen, Trifluormethyl, Hydroxy, das in freier Form vorliegt oder mit einer Säure mit 1 bis 6 Kohlenstoffatomen verestert ist, ausgewählt ist, oder mit einer Carboxygruppe substituiert sein können, die frei vorliegt oder mit einem niederen Alkohol mit 1 bis 6 Kohlenstoffatomen verestert ist;
R' eine Hydroxygruppe oder eine Estergruppe der Formel 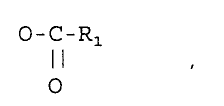 worin R₁ eine gesättigte oder ungesättigte aliphatische Gruppe mit 1 bis 18 Kohlenstoffatomen bedeutet;
R'' Wasserstoff, Hydroxy oder eine Estergruppe der Formel 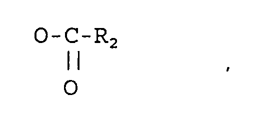 worin R₂ eine aliphatische gesättigte oder ungesättigte Gruppe mit 1 bis 18 Kohlenstoffen bedeutet, die mit der Gruppe R₁ identisch oder davon verschieden ist.

2. Verwendung nach Anspruch 1, dadurch gekennzeichnet, daß das Salicylsäurederivat unter den Derivaten der Formel I ausgewählt ist, worin R'' Wasserstoff bedeutet.

3. Verwendung nach Anspruch 2, dadurch gekennzeichnet, daß das Salicylsäurederivat unter 5-(n-Octanoyl)-salicylsäure, 5-(n-Decanoyl)-salicylsäure, 5-(n-Dodecanoyl)-salicylsäure, 5-(n-Octyl)-salicylsäure, 5-(n-Heptyloxy)-salicylsäure und 4-(n-Heptyloxy)-salicylsäure ausgewählt ist.

4. Verwendung nach Anspruch 3, dadurch gekennzeichnet, daß die Salicylsäure die 5-(n-Octanoyl)-salicylsäure ist.

5. Verwendung nach Anspruch 1, dadurch gekennzeichnet, daß das Salicylsäurederivat unter den Derivaten der Formel I ausgewählt ist, worin R oder R'' Hydroxy bedeutet.

6. Verwendung nach Anspruch 5, dadurch gekennzeichnet, daß das Salicylsäurederivat unter Gentisinsäure und 2,3-Dihydroxybenzoesäure ausgewählt ist.

7. Verwendung nach einem der vorhergehenden Ansprüche, dadurch gekennzeichnet, daß das Salicylsäurederivat in Form eines Salzes mit einer anorganischen oder organischen Base vorliegt.

8. Verwendung nach einem der vorhergehenden Ansprüche, dadurch gekennzeichnet, daß das Salicylsäurederivat oder sein Salz in einer Konzentration von 0,001 bis 20 Gew.-%, bezogen auf das Gesamtgewicht der Zusammensetzung, vorliegt.

9. Verwendung nach einem der vorhergehenden Ansprüche, dadurch gekennzeichnet, daß die Zusammensetzung eine wäßrige, ölige oder wäßrig-alkoholische Lösung, Wasser-in-Öl-Emulsion, Öl-in-Wasser-Emulsion, Mikroemulsion, wäßriges Gel, öliges Gel, wasserfreies Gel, Serum oder Vesikeldispersion ist.

10. Kosmetisches Verfahren zur Vorbeugung und/oder Behandlung von Streifen, das darin besteht, auf die Haut eine Zusammensetzung aufzutragen, die ein Salicylsäurederivat der Formel I oder ein Salz dieser Derivate enthält: 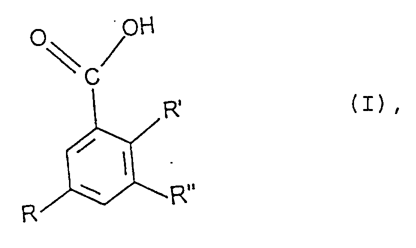 worin bedeuten:
R eine Hydroxygruppe, eine gesättigte, geradkettige, verzweigte oder cyclische aliphatische Gruppe, Alkoxygruppe, Estergruppe oder Acylgruppe und eine ungesättigte Gruppe, die eine oder mehrere ggf. konjugierte Doppelbindungen aufweist, wobei die Gruppen 2 bis 22 Kohlenstoffatome aufweisen und mit mindestens einem Substituenten, der unter Halogen, Trifluormethyl, Hydroxy, das in freier Form vorliegt oder mit einer Säure mit 1 bis 6 Kohlenstoffatomen verestert vorliegt, ausgewählt ist, oder mit einer Carboxygruppe substituiert sein können, die frei vorliegt oder mit einem niederen Alkohol mit 1 bis 6 Kohlenstoffatomen verestert ist;
R' eine Hydroxygruppe oder eine Estergruppe der Formel 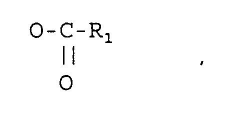 worin R₁ eine gesättigte oder ungesättigte aliphatische Gruppe mit 1 bis 18 Kohlenstoffatomen bedeutet;
R'' Wasserstoff, Hydroxy oder eine Estergruppe der Formel 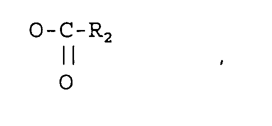 worin R₂ eine aliphatische gesättigte oder ungesättigte Gruppe mit 1 bis 18 Kohlenstoffen bedeutet, die mit der Gruppe R₁ identisch oder davon verschieden ist.

11. Verfahren nach Anspruch 10, dadurch gekennzeichnet, daß das Salicylsäurederivat unter den Derivaten der Formel I ausgewählt ist, worin R'' Wasserstoff bedeutet.

12. Verfahren nach Anspruch 11, dadurch gekennzeichnet, daß das Salicylsäurederivat unter 5-(n-Octanoyl)-salicylsäure, 5-(n-Decanoyl)-salicylsäure, 5-(n-Dodecanoyl)-salicylsäure, 5-(n-Octyl)-salicylsäure, 5-(n-Heptyloxy)-salicylsäure und 4-(n-Heptyloxy)-salicylsäure ausgewählt ist.

13. Verwendung nach Anspruch 12, dadurch gekennzeichnet, daß das Salicylsäurederivat die 5-(n-Octanoyl)-salicylsäure ist.

14. Verfahren nach Anspruch 10, dadurch gekennzeichnet, daß das Salicylsäurederivat unter den Derivaten der Formel I ausgewählt ist, worin R oder R'' eine Hydroxygruppe bedeutet.

15. Verfahren nach Anspruch 14, dadurch gekennzeichnet, daß das Salicylsäurederivat unter Gentisinsäure und 2,3-Dihydroxybenzoesäure ausgewählt ist.
